# EUROPEAN PATENT APPLICATION

(11) **EP 4 843 618 A2**
(43) Date of publication of application: **25.11.2026**
(21) Application number: 26178793.1
(22) Date of filing: 14.05.2026
(51) Int. Cl.: A61N 5/10

(54) **SYSTEMS AND METHODS FOR INTEGRATING TREATMENT OUTCOMES WHEN GENERATING RADIOTHERAPY TREATMENT PLANS**

(30) Priority: 22.05.2025 US 202519216491
(71) Applicant: Siemens Healthineers International AG, 6312 Steinhausen (CH)
(72) Inventor: CZEIZLER, Elena, 00100 Helsinki (FI); SACKETT, Jonathan, Castle Pines, Colorado, 80108 (US)
(74) Representative: Mathisen & Macara LLP

(57) **Abstract**

Provided herein are systems (100) for integrating treatment outcomes when generating radiotherapy treatment plans. In some examples, a system (100) can be configured to determine (202) a first dose distribution indicating an instant amount of dose targeting a planning target volume (PTV) of a patient and obtain (204) a second dose distribution indicating a predetermined amount of dose that was delivered, or is to be delivered, to the patient. The system (100) can determine (206) a first result based on the first dose distribution and a second result based on the second dose distribution, each indicating a degree of on-target effect and an off-target effect. In some examples, the system (100) can then compare (208) the first result and the second result to determine a difference between the first result and the second result, and update (210) one or more treatment plan parameters based on the difference between the first result and the second result.

## Description

### TECHNICAL FIELD

This application relates generally to systems and methods for integrating treatment outcomes when generating radiotherapy treatment plans and, in some embodiments, to systems and methods for integrating treatment outcomes when generating radiotherapy treatment plans using machine learning-based techniques.

### BACKGROUND

Radiation therapy (also referred to as "radiotherapy" or "RT") involves the delivery of radiation (energy) to targets within the body of a patient during treatment. For example, a linear accelerator (LINAC) can be configured to move relative to a patient in accordance with an RT treatment plan to multiple control points and deliver energy to target tissue (e.g., tumors, lesions, etc.) to treat cancer located within the body of the patient. This can allow for the targeted delivery of energy to the target tissue, with the goal of reducing or eliminating the target tissue with minimal off-target effects on the surrounding healthy tissue.

But conventional methods for generating treatment plans can be technically challenging for a variety of reasons. First, by virtue of the complexity of radiotherapy plans, the patients' responses to them and the heterogeneity of both patient populations as well as the planning approaches used in different clinical practices, it is difficult to develop universally applicable approaches that can be used to build optimized treatment plans. Second, the limited availability of large, high-quality datasets and the need for extensive computational resources make it difficult to accurately test treatment planning systems that generate these treatment plans. As a result, conventional treatment planning systems can struggle to accurately predict patient outcomes due to their reliance on complex algorithms and iterative processes that require significant computational resources. And these iterative processes can, when implemented, involve the dedication of further processing power and memory to generate effective treatment plans that balance the competing objectives of tumor coverage and organ sparing.

### SUMMARY

In accordance with a first facet of the invention, there is provided a system for efficient policy generation using reinforcement learning to optimize radiotherapy treatment plan generation, as defined by claim 1.

In accordance with a second facet of the invention, there is provided a system as defined by claim 4.

In accordance with a third facet of the invention, there is provided a method as defined by claim 9.

In accordance with a fourth facet of the invention, there is provided a non-transitory computer-readable medium as defined by claim 14.

Optional features are defined by the dependent claims.

For the aforementioned reasons, there is a need for systems and methods that allow for integrating treatment outcomes when generating radiotherapy treatment plans.

As the complexity of radiation therapy techniques increases, so does the computational burden associated with treatment planning. Some treatment planning systems (TPSs) may be configured to allocate significant computing resources to perform iterative optimization calculations. However, even with these resources, the iterative optimization of treatment plans to arrive at an optimized treatment plan can lead to excessive computing resource consumption as well as prolonged planning times and potential treatment delays. For example, some optimization algorithms can struggle to efficiently navigate vast search spaces of possible treatment parameters and can sometimes settle on suboptimal solutions (sometimes referred to as a local minima or maxima within a given search space). Moreover, the growing reliance on network-based solutions for data transfer introduces additional challenges in terms of speed and system interoperability. While some radiotherapy treatment planning platforms have improved the quality and precision of radiotherapy, they can face challenges as the need for additional computing resources grows. As a result, the focus on optimizing proxy metrics such as dosimetric features, while important, may not always translate directly to maximized patient outcomes, highlighting a growing need for innovative approaches that more directly link treatment planning to improved clinical results. The techniques included in the methods and systems discussed herein address the challenges associated with optimization of treatment plans by allowing for the integration of possible treatment outcomes when generating radiotherapy treatment plans.

By implementing some or all of the techniques described herein, multiple technical and clinical benefits can be realized. First, integrating treatment outcomes into policy generation using a reinforcement learning agent can significantly enhance the effectiveness and personalization of treatment plans. For example, the reinforcement learning agent can continuously learn from the treatment outcomes of patients, such as the presence and severity of side effects (also referred to as off-target effects where contextually appropriate), to refine its policy. In this example, the agent can adjust treatment plan parameters in real-time, ensuring that the plans are tailored to the individual needs and responses of each patient. In some examples, this approach can lead to more accurate predictions of patient responses to treatments, reducing the likelihood of adverse off-target effects and improving overall patient outcomes.

In another example, the integration of treatment outcomes can enable the reinforcement learning agent to identify patterns and correlations that may not be apparent through traditional methods, leading to more informed decision-making and optimized treatment strategies. The reinforcement learning agent can also adapt to new data and evolving medical knowledge, ensuring that the policy used to generate the treatment plans remain up-to-date and relevant. In some examples, this can result in more efficient use of healthcare resources (e.g., power and computing resources reserved for treatment plan generation and medical device (LINAC) operation), as treatments can be adjusted based on real-time feedback, reducing the need for trial- and-error approaches and minimizing unnecessary interventions. In this example, the reinforcement learning agent can also facilitate personalized medicine by considering the unique characteristics and treatment outcomes of each patient, leading to more effective and targeted treatments that can extend a patient's probable lifespan when confronted with a disease while minimizing the side effects realized by the patient as a result of radiotherapy treatment.

Using policies instead of conventional treatment planning systems can also reduce processing resource consumption and/or accelerate conventional plan generation processes, as the reinforcement learning agent can learn to use policies that streamline the decision-making processes. In an example, conventional systems often rely on complex algorithms and extensive computations to generate treatment plans, which can be resource-intensive and very time-consuming. In an optional example according to the present disclosure, a reinforcement learning agent can use and/or configure policies to make decisions more efficiently, bypassing the need for many rounds of repetitive calculations when optimizing a given treatment plan for a patient using conventional approaches. In some examples, the reinforcement learning agent can quickly reference these policies to determine the optimal treatment plan parameters at a given stage when generating a treatment plan, significantly reducing the computational load. In another example, the agent can continuously update and refine its policies based on new treatment outcomes, ensuring that the decision-making process remains efficient and up-to-date. In this example, this approach can lead to faster processing times and lower energy consumption, making the system more sustainable and cost-effective.

In some examples, the streamlined decision-making process can also enhance the scalability of the system, allowing it to handle a larger number of patients without a corresponding increase in processing power and clinical skilled resources (e.g. medical physicists or dosimetrists) requirements. In one example, the reinforcement learning agent can allocate computational resources more effectively, prioritizing critical tasks and minimizing unnecessary computations. In another example, memory can be conserved by storing only the most relevant data and outcomes, rather than extensive historical records. In this example, treatment plans can be developed more quickly, as the agent can rapidly process and integrate new information to generate optimized plans.

In an embodiment a system can include one or more processors configured to determine a first dose distribution indicating an instant amount of dose to be delivered to a planning target volume (PTV) of a patient at a first point in time based on one or more treatment plan parameters that control operation of a linear accelerator (LINAC). The one or more processors can be configured to obtain a second dose distribution indicating a predetermined amount of dose delivered to the patient during treatment at a second point in time prior to the first point in time. The one or more processors can be configured to determine a first result based on the first dose distribution and a second result for the second dose distribution. The first result can indicate a first degree of an on-target effect and a first off-target effect, and the second result indicating a second degree of the on-target effect and a second off-target effect. The one or more processors can compare the first result and the second result to determine a difference between the first result and the second result. The one or more processors can execute an update to the one or more treatment plan parameters based on the difference between the first result and the second result, wherein the update is configured to adjust operation of a linear accelerator (LINAC) in accordance with the one or more treatment plan parameters.

In some examples, the one or more processors can be further configured to obtain a profile representing a patient in a predetermined state having tissue to be targeted using the treatment plan parameters. The one or more processors can be further configured to determine the one or more treatment plan parameters based on the profile of the patient in the predetermined state. In some aspects, the profile can include one or more computed tomography (CT) images, one or more field geometry parameters, and/or one or more prescription levels indicating a target dose to deliver to the PTV of the patient.

In some aspects, the one or more processors configured to compare the first result and the second result can be configured to provide the first dose distribution to a machine learning model to cause the machine learning model to generate a first outcome prediction indicating a probability associated with the first off-target effect. The one or more processors can be configured to provide the second dose distribution to the machine learning model to cause the machine learning model to generate a second outcome prediction indicating a probability associated with the second off-target effect. In some aspects, the one or more processors can be configured to compare the first outcome prediction to the second outcome prediction to determine the difference between the first result and the second result. The difference can indicate a degree according to which the first result is different from the second result.

In some aspects, the one or more processors configured to provide the first dose distribution to the machine learning model can be configured to provide the first dose distribution to the machine learning model to cause the machine learning model to generate a first outcome prediction indicating a first probability that the patient will experience one or more off-target effects. The one or more processors can be configured to provide the second dose distribution to the machine learning model to cause the machine learning model to generate a second outcome prediction indicating a second probability that the patient will experience the one or more off-target effects.

In some aspects, the one or more processors can be further configured to provide the first dose distribution, the second dose distribution, the first result, and the second result to a reinforcement learning (RL) agent to cause the RL agent to determine the update to the one or more treatment plan parameters. The one or more processors can be further configured to determine that updating the treatment plan parameters causes one or more of the treatment plan parameters to satisfy a convergence threshold, and determine that the RL agent converged based on the treatment plan parameters satisfying the convergence threshold. In some aspects, the one or more processors can be further configured to determine that one or more of the treatment plan parameters do not satisfy a convergence threshold based on updating the treatment plan parameters. In response to determining that the one or more treatment plan parameters do not satisfy the convergence threshold, the one or more processors can be configured to cause the RL agent to update a policy to adjust outputs of the policy based on the treatment plan parameters not satisfying the convergence threshold.

In some aspects, the one or more processors configured to determine the first dose distribution indicating an instant amount of dose delivered to a planning target volume (PTV) of a patient based on one or more treatment plan parameters can be configured to determine the first dose distribution indicating an instant amount of dose delivered to a planning target volume (PTV) of a patient based on a cost function establishing the one or more treatment plan parameters.

In another embodiment, a method can include determining, by one or more processors, a first dose distribution indicating an instant amount of dose to be delivered to a planning target volume (PTV) of a patient at a first point in time based on one or more treatment plan parameters that control operation of a linear accelerator (LINAC). The method can include obtaining, by the one or more processors, a second dose distribution indicating a predetermined amount of dose delivered to the patient during treatment at a second point in time prior to the first point in time. In some aspects the method can include determining, by the one or more processors, a first result based on the first dose distribution and a second result for the second dose distribution, the first result indicating a first degree of an on-target effect and a first off-target effect, and the second result indicating a second degree of an on-target effect and a second off-target effect. In some aspects, the method can include comparing, by the one or more processors, the first result and the second result to determine a difference between the first result and the second result. In some aspects, the method can include executing, by the one or more processors, an update to the one or more treatment plan parameters based on the difference between the first result and the second result. The update can be configured to adjust operation of a linear accelerator (LINAC) in accordance with the one or more treatment plan parameters.

In some aspects, the method can further include obtaining, by the one or more processors, a profile representing a patient in a predetermined state having tissue to be targeted using the treatment plan parameters. The method can include determining, by the one or more processors, the one or more treatment plan parameters based on the profile of the patient in the predetermined state. In some aspects, the profile can include one or more computed tomography (CT) images, one or more field geometry parameters, and/or one or more prescription levels indicating a target dose to deliver to the PTV of the patient.

In some aspects, comparing the first result and the second result can include providing, by the one or more processors, the first dose distribution to a machine learning model to cause the machine learning model to generate a first outcome prediction indicating a probability associated with the first off-target effect. The method can include providing, by the one or more processors, the second dose distribution to the machine learning model to cause the machine learning model to generate a second outcome prediction indicating a probability associated with the second off-target effect. In some aspects, the method can include comparing, by the one or more processors, the first outcome prediction to the second outcome prediction to determine the difference between the first result and the second result. The difference can indicate a degree according to which the first result is different from the second result.

In some aspects, providing the first dose distribution to the machine learning model can include providing, by the one or more processors, the first dose distribution to the machine learning model to cause the machine learning model to generate a first outcome prediction indicating a first probability that the patient will experience one or more off-target effects, and providing, by the one or more processors, the second dose distribution to the machine learning model to cause the machine learning model to generate a second outcome prediction indicating a second probability that the patient will experience the one or more off-target effects. In some aspects, the method can further include providing, by the one or more processors, the first dose distribution, the second dose distribution, the first result, and the second result to a reinforcement learning (RL) agent to cause the RL agent to determine the update to the one or more treatment plan parameters.

In at least some aspects, the method can further include determining, by the one or more processors, that updating the treatment plan parameters causes one or more of the treatment plan parameters to satisfy a convergence threshold, and determining, by the one or more processors, that the RL agent converged based on the treatment plan parameters satisfying the convergence threshold. In some aspects, determining the first dose distribution indicating an instant amount of dose delivered to a planning target volume (PTV) of a patient based on one or more treatment plan parameters can include determining, by the one or more processors, the first dose distribution indicating an instant amount of dose delivered to a planning target volume (PTV) of a patient based on a cost function establishing the one or more treatment plan parameters.

In yet another embodiment, a non-transitory computer-readable medium can store instructions thereon that, when executed by one or more processors, cause the one or more processors to determine a first dose distribution indicating an instant amount of dose to be delivered to a planning target volume (PTV) of a patient at a first point in time based on one or more treatment plan parameters that control operation of a linear accelerator (LINAC). The instructions can cause the one or more processors to obtain a second dose distribution indicating a predetermined amount of dose delivered to the patient during treatment at a second point in time prior to the first point in time. In some aspects, the instructions can cause the one or more processors to determine a first result based on the first dose distribution and a second result for the second dose distribution. The first result can indicate a first degree of an on-target effect and a first off-target effect, and the second result can indicate a second degree of the on-target effect and a second off-target effect. In at least some aspects, the instructions can cause the one or more processors to compare the first result and the second result to determine a difference between the first result and the second result. The instructions can cause the one or more processors to execute an update to the one or more treatment plan parameters based on the difference between the first result and the second result. The update can be configured to adjust operation of a linear accelerator (LINAC) in accordance with the one or more treatment plan parameters.

In some aspects, the instructions can further cause the one or more processors to obtain a profile representing a patient in a predetermined state having tissue to be targeted using the treatment plan parameters, and determine the one or more treatment plan parameters based on the profile of the patient in the predetermined state. In some aspects, the profile can include one or more computed tomography (CT) images, one or more field geometry parameters, and/or one or more prescription levels indicating a target dose to deliver to the PTV of the patient.

### Other examples - policy generation

Optionally, a method may comprise efficient policy generation using reinforcement learning to optimize radiotherapy treatment plan generation, the method comprising:
obtaining a profile of a patient in a predetermined state, the profile of the patient indicating one or more locations for tissue to be targeted;
providing the profile of the patient as input to a policy configured using a reinforcement learning (RL) agent to cause the policy to generate an output in accordance with one or more treatment plan parameters,
wherein the RL agent is configured to iteratively update the policy based on dose distributions and corresponding on-target and off-target effects resulting from targeting tissue during radiotherapy treatment;
and optionally controlling operation of a medical device based on the output of the policy.

Optionally, the off-target effects comprise one or more of: rectal bleeding, radiation pneumonitis, or xerostomia and wherein the on-target effects comprise one or more of: local control of a tumor or tumor recurrence.

Optionally, the method comprises:
generating one or more control signals to control operation of the medical device in accordance with the output of the policy,
and optionally controlling operation of the medical device based on the one or more control signals.

Optionally, the medical device comprises a linear accelerator (LINAC), and optionally wherein the method further comprises delivering dose, by the medical device, to at least a portion of the tissue to be targeted based on the output of the policy.

Optionally, a non-transitory computer-readable medium may store instructions thereon that, when executed by one or more processors, cause the one or more processors to perform a method of efficient policy generation using reinforcement learning to optimize radiotherapy treatment plan generation, the method comprising:
obtaining a profile of a patient in a predetermined state, the profile of the patient indicating one or more locations for tissue to be targeted;
providing the profile of the patient as input to a policy configured using a reinforcement learning (RL) agent to cause the policy to generate an output in accordance with one or more treatment plan parameters,
wherein the RL agent is configured to iteratively update the policy based on dose distributions and corresponding on-target and off-target effects resulting from targeting tissue during radiotherapy treatment; and
controlling operation of a medical device based on the output of the policy.

Optionally, the off-target effects comprise one or more of: rectal bleeding, radiation pneumonitis, or xerostomia and wherein the on-target effects comprise one or more of: local control of a tumor or tumor recurrence.

Optionally, the non-transitory computer-readable medium may store instructions thereon that, when executed by one or more processors, may cause the one or more processors to:
generate one or more control signals to control operation of the medical device in accordance with the output of the policy, and
control operation of the medical device based on the one or more control signals.

Optionally, the non-transitory computer-readable medium may store instructions thereon that, when executed by one or more processors, may cause the one or more processors to:
cause the medical device comprising a LINAC to deliver dose to at least a portion of the tissue to be targeted based on the output of the policy.

### Other examples - updating treatment plan parameters

Optionally, the method may further comprise:
determining that one or more of the treatment plan parameters do not satisfy a convergence threshold based on updating the treatment plan parameters; and
in response to determining that the one or more treatment plan parameters do not satisfy the convergence threshold, causing the RL agent to update a policy to adjust outputs of the policy based on the treatment plan parameters not satisfying the convergence threshold.

Optionally, the non-transitory computer-readable medium may store instructions thereon that, when executed by one or more processors, may cause the one or more processors to:
provide the first dose distribution to a machine learning model to cause the machine learning model to generate a first outcome prediction indicating a probability associated with the first off-target effect and the first on-target effect;
provide the second dose distribution to the machine learning model to cause the machine learning model to generate a second outcome prediction indicating a probability associated with the second off-target effect; and
compare the first outcome prediction to the second outcome prediction to determine the difference between the first result and the second result, where the difference indicates a degree according to which the first result is different from the second result.

Optionally, the non-transitory computer-readable medium may store instructions thereon that, when executed by one or more processors, may cause the one or more processors to:
provide the first dose distribution to the machine learning model to cause the machine learning model to generate a first outcome prediction indicating a first probability that the patient will experience one or more off-target effects and one or more on-target effects, and
provide the second dose distribution to the machine learning model to cause the machine learning model to generate a second outcome prediction indicating a second probability that the patient will experience the one or more off-target effects.

Optionally, the non-transitory computer-readable medium may store instructions thereon that, when executed by one or more processors, may cause the one or more processors to:
provide the first dose distribution, the second dose distribution, the first result, and the second result to a reinforcement learning (RL) agent to cause the RL agent to determine the update to the one or more treatment plan parameters.

Optionally, the non-transitory computer-readable medium may store instructions thereon that, when executed by one or more processors, may cause the one or more processors to:
determine that updating the treatment plan parameters causes one or more of the treatment plan parameters to satisfy a convergence threshold; and
determine that the RL agent converged based on the treatment plan parameters satisfying the convergence threshold.

Optionally, the non-transitory computer-readable medium may store instructions thereon that, when executed by one or more processors, may cause the one or more processors to:
determine that one or more of the treatment plan parameters do not satisfy a convergence threshold based on updating the treatment plan parameters; and
in response to determining that the one or more treatment plan parameters do not satisfy the convergence threshold, cause the RL agent to update a policy to adjust outputs of the policy based on the treatment plan parameters not satisfying the convergence threshold.

Optionally, the non-transitory computer-readable medium may store instructions thereon that, when executed by one or more processors, may cause the one or more processors to:
determine the first dose distribution indicating an instant amount of dose delivered to a planning target volume (PTV) of a patient based on a cost function establishing the one or more treatment plan parameters.

### BRIEF DESCRIPTION OF THE DRAWINGS

**FIG. 1** illustrates a diagram of a system for integrating treatment outcomes when generating radiotherapy treatment plans, according to an embodiment.
**FIG. 2** illustrates a flow diagram of a process for integrating treatment outcomes during the training of a reinforcement learning agent for radiotherapy treatment planning, according to an embodiment.
**FIG. 3** illustrates an implementation diagram of a process for learning a treatment policy by training a reinforcement learning agent for radiotherapy treatment plan generation, according to an embodiment.
**FIG. 4** illustrates an implementation diagram of a process for using a policy trained using a reinforcement learning agent when generating radiotherapy treatment plans, according to an embodiment.

### DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are configured to be considered within the scope of the subject matter disclosed herein. Other embodiments can be used, or other changes can be made without departing from the scope of the present disclosure. The illustrative embodiments described in the detailed description are not meant to be limiting of the subject matter presented.

Some approaches to radiotherapy treatment planning are faced with significant challenges in directly optimizing patient outcomes, as these approaches can rely on dosimetric proxies rather than maximizing tumor control and healthy tissue sparing. For example, treatment planning systems (TPSs) can be configured to achieve predetermined dosimetric goals, such as mean dose and D98 for targets, which serve as indirect indicators of clinical outcomes. But these indirect indicators may not reflect the complexities of patient responses by specific patients as they are often derived from population-level data in clinical protocols and do not account for unique patient characteristics, potentially leading to suboptimal treatment plans. Additionally, the multistep process of interpreting clinical goals, translating them into TPS objectives, and optimizing treatment plans based on these objectives can introduce variability and inconsistency in plan quality. For example, treatment planning systems can struggle to formulate comprehensive optimization objectives that capture relevant clinical goals, resulting in plans that may meet specified objectives but do not optimize for other aspects of a patient's short- to long-term treatment outcomes. Additionally, reliance on rigid cost function definitions can limit the ability to represent complex trade-offs between tumor control and normal tissue sparing, contributing to significant variability in plan quality across different planners and institutions.

In some examples, radiotherapy treatment plans can be evaluated using tumor control probability (TCP) and normal tissue complication probability (NTCP) models. However, these models can face significant challenges as well that can make it difficult to establish their direct implementation in TPSs and limit their ability to provide truly personalized treatment optimization. Some radiobiological models for TCP and NTCP have a narrow window of validity, often derived from specific clinical scenarios or datasets, which can restrict their generalizability to diverse patient populations and treatment regimens. These models can rely on dose-volume histograms (DVHs) and basic fractionation information, oversimplifying the complex 3D dosimetric and anatomical information available and potentially overlooking important spatial relationships and dose gradients that could impact treatment outcomes. Furthermore, some of these models may not incorporate a wide range of potentially relevant patient-specific factors, including anatomical relationships, dose gradients, patient demographics, genomic and genetic information, and functional imaging data. This limitation can make it difficult to establish a patient-specific approach to treatment plan generation and optimization.

The integration of TCP/NTCP models into treatment planning systems can also be hindered by the complexity of these models, the uncertainties associated with their predictions, and the lack of standardization across different treatment sites and endpoints. Additionally, some more sophisticated TCP/NTCP models can use significant computational resources, potentially slowing down the treatment planning process if implemented directly into the TPS.

Due to these challenges, treatment planning can rely on DVH threshold heuristics derived from clinical trial and retrospective data. These heuristics can provide a set of population-level standard objectives for treatment planning. But this approach may not be able to account for individual variations in tumor biology, normal tissue radiosensitivity, or other patient-specific factors that could influence treatment outcomes.

The present disclosure describes the techniques that involve incorporating expected patient treatment outcomes into the treatment planning process so as to optimize treatment outcomes for individual patients. In some examples, a model (also referred to as a machine learning model or a deep learning-based model) can be configured to predict treatment outcomes for specific individuals, such as tumor control and/or healthy tissue sparing. These models can also be configured together with explainable AI approaches like Gradient-weighted Class Activation Mapping (Grad-CAM), which can create heatmaps to indicate the most influential features from input images. This information can allow for integration of outcome prediction models into the treatment planning process, emphasizing regions where dose distribution can be adjusted for improved tumor control and/or healthy tissue sparing.

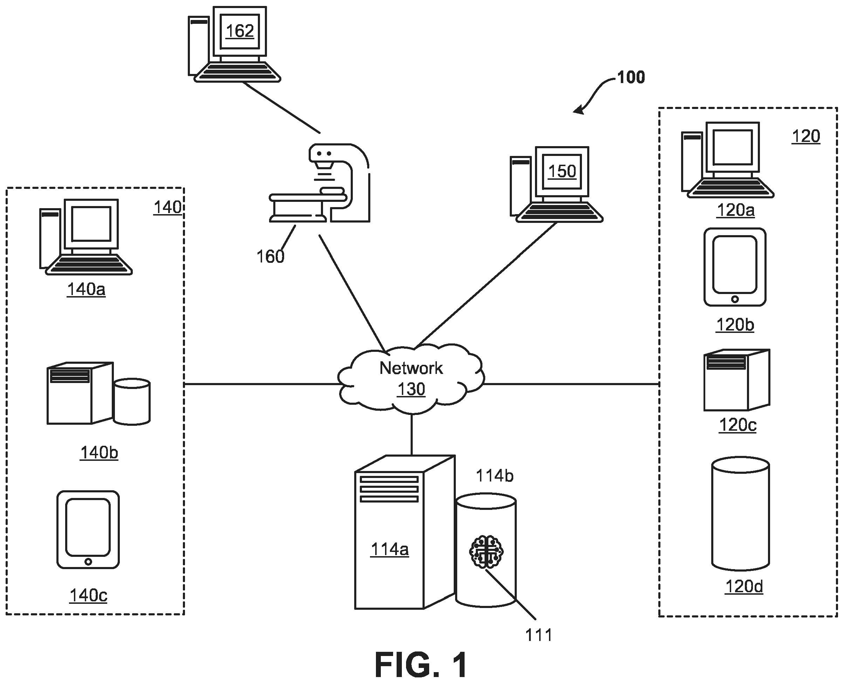

**FIG. 1** illustrates components of a system **100** for integrating treatment outcomes when generating radiotherapy treatment plans, according to an embodiment. The system **100** can include an analytics server **114a,** system database **114b,** a machine learning model **111,** electronic data sources **120a-d** (each referred to individually as an electronic data source **120** and collectively electronic data sources **120,** unless stated otherwise), end-user devices **140a-c** (each referred to individually as an end user device **140** and collectively as end-user devices **140,** unless stated otherwise), an administrator computing device **150,** a medical device **160,** and medical device computer(s) **162.** Various components depicted in **FIG. 1** can belong to a radiotherapy clinic at which patients can receive radiotherapy treatment, in some cases via one or more radiotherapy machines (e.g., medical device **160**) located within the clinic. The system **100** is not confined to the components described herein and can include additional or other components, not shown for brevity, which are configured to be considered within the scope of the embodiments described herein.

The above-mentioned components can be connected to each other through a network **130.** Examples of the network **130** can include, but are not limited to, private or public local-area-networks (LAN), wireless LAN (WLAN) networks, metropolitan area networks (MAN), wide-area networks (WAN), and the Internet. The network **130** can include wired or wireless communications according to one or more standards or via one or more transport mediums. The communication over the network **130** can be performed in accordance with various communication protocols such as Transmission Control Protocol and Internet Protocol (TCP/IP), User Datagram Protocol (UDP), and IEEE communication protocols. In one example, the network **130** can include wireless communications according to Bluetooth specification sets or another standard or proprietary wireless communication protocol. In another example, the network **130** can also include communications over a cellular network, including, e.g., a GSM (Global System for Mobile Communications), CDMA (Code Division Multiple Access), and EDGE (Enhanced Data for Global Evolution) network.

The analytics server **114a** can be any computing device comprising a processor and non-transitory machine-readable storage capable of executing the various tasks and processes described herein. The analytics server **114a** can employ various processors such as central processing units (CPU) and graphics processing unit (GPU), among others. Non-limiting examples of such computing devices can include workstation computers, laptop computers, server computers, and the like. While the system **100** includes a single analytics server **114a,** the analytics server **114a** can include any number of computing devices operating in a distributed computing environment, such as a cloud environment.

The analytics server **114a** can generate and display an electronic platform configured to use a machine learning model **111** for receiving patient information and inputs from users (e.g., clinicians) such as utility functions and updated utility functions described herein and outputting the results of execution of the machine learning model **111.** The electronic platform can include graphical user interfaces (GUI) displayed by display devices of one or more electronic data sources **120,** the end-user devices **140,** the medical device **160,** or the administrator computing device **150.** An example of the electronic platform generated and hosted by the analytics server **114a** can be a web-based application or a website configured to be displayed on different electronic devices, such as mobile devices, tablets, personal computers, and the like.

The information displayed by the electronic platform can include, for example, input elements to receive data associated with a patient being treated, synchronize one or more sensors, and display results of predictions produced by the machine learning model **111.** For instance, the analytics server **114a** can execute the machine learning model **111** (e.g., a system such as a treatment planner that is configured or trained to generate beam configurations that are usable to configure the medical device **160** when treating a patient, as described herein). The analytics server **114a** can then display the results for a clinician or directly revise one or more operational attributes of the medical device **160.**

The electronic data sources **120** can be any computing device comprising a processor and non-transitory machine-readable storage capable of executing the various tasks and processes described herein. For example, the electronic data sources **120** can represent various computing devices that contain, retrieve, or access data associated with a medical device **160,** such as data associated with operational information of currently or previously performed radiotherapy treatments (e.g., electronic log files or electronic configuration files), data associated with current or previously monitored patients (e.g., computed tomography (CT) scans, magnetic resonance imaging (MRI) scans, tumor locations, deformation information, or the like) or participants in a study, or the like. For instance, the analytics server **114a** can use the clinic computer **120a,** medical professional device **120b,** server **120c** (associated with a clinician or a clinic), and database **120d** (associated with the clinician or the clinic) to retrieve/receive data associated with the medical device **160.** The analytics server **114a** can retrieve the data from the end-user devices **140,** generate a dataset, and use the dataset to configure the machine learning model **111** (e.g., models implemented by the machine learning model **111** or the like). The analytics server **114a** can execute various algorithms to translate raw data received/retrieved from the electronic data sources **120** into machine-readable objects that can be stored and processed by other analytics processes as described herein.

End-user devices **140** can be any computing device comprising a processor and a non-transitory machine-readable storage medium capable of performing the various tasks and processes described herein. Non-limiting examples of an end-user device **140** can be a workstation computer, laptop computer, tablet computer, or server computer. In operation, various users such as clinicians as described herein can use end-user devices **140** to access the GUI operationally managed by the analytics server **114a** or otherwise the results of the execution of the machine learning model **111.** Specifically, the end-user devices **140** can include clinic computer **140a,** clinic server **140b,** and a medical professional device **140c.** Even though referred to herein as "end-user" devices, these devices cannot always be operated by end-users. For instance, the clinic server **140b** cannot be directly used by an end user. However, the results stored on the clinic server **140b** can be used to populate various GUIs accessed by an end user via the medical professional device 140c. In some embodiments, the end-user device **140** can be associated with one or more clinicians that are associated with the generation of one or more treatment plans (e.g., involved in preparing the one or more treatment plans) for patients.

The administrator computing device **150** can represent a computing device operated by a system administrator. The administrator computing device **150** can be configured to display radiotherapy treatment attributes generated by the analytics server **114a** (e.g., various analytics metrics determined during training of one or more machine learning models or systems); monitor various treatment planning systems utilized by the analytics server **114a,** electronic data sources **120,** or end-user devices **140**; review feedback; or facilitate training or retraining (calibration) of the machine learning model **111** that are maintained by the analytics server **114a.**

In some embodiments, the medical device **160** can be a diagnostic imaging device or a treatment delivery device (also referred to as a radiotherapy system). For example, the medical device **160** can include one or more computed tomography (CT) scanners such as a cone-beam CT (CBCT) scanner, a linear accelerator (LINAC) such as the Varian^{®} TrueBeam^{®} LINAC, proton beam therapy systems (referred to as proton beam systems) that uses accelerated protons to deliver precise radiotherapy to tumors, or other similar devices configured to transmit energy toward targeted tissue (referred to as gross target volumes) associated with a patient and, in some cases, measure the energy transferred toward the targeted tissue. The medical device **160** can also include one or more sensors configured to monitor the patient being treated. That is, the medical device **160** or the analytics server **114a** can be communicating with various sensors that can monitor a patient's external biological signals. Non-limiting examples of the sensors can include three-dimensional (3D) surfacing mechanisms, and optical (or other) sensors configured to monitor the patient's movements (e.g., how the patient is moving or breathing). In some embodiments, the medical device **160** can receive data associated with a treatment plan from the medical device computer(s) **162** that cause the medical device **160** to operate in accordance with the treatment plan.

The machine learning model **111** can be stored in the system database **114b.** The machine learning model **111** can be trained using data received/retrieved from the electronic data sources **120** and can be executed using data received from the end-user devices, the medical device **160,** or the sensor **163.** In some embodiments, the machine learning model **111** can reside within a data repository local or specific to a clinic. In various embodiments, the machine learning model **111** can use one or more deep learning engines to develop a treatment plan for a patient using radiotherapy. For instance, the analytics server **114a** can transmit patient attributes from the sensor **163** and execute the machine learning model **111** accordingly. The analytics server **114a** can then display the results on one or more end-user devices **140.** In some embodiments, the analytics server **114a** can change one or more configurations of the medical device **160** based on the results predicted by the machine learning model **111.**

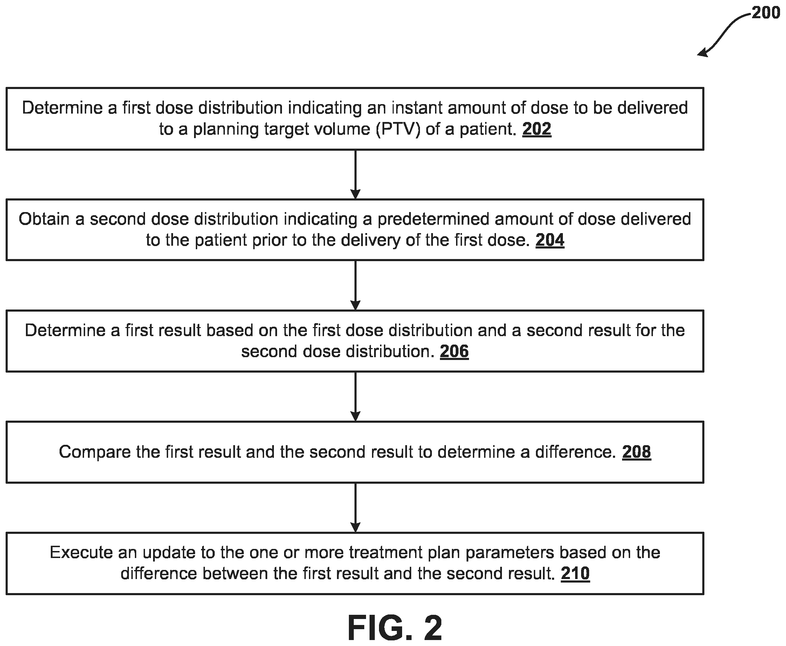

Referring to **FIG. 2****,** illustrated is a flow diagram of a process **200** for integrating treatment outcomes during the training of a reinforcement learning agent for radiotherapy treatment planning, according to an embodiment. The process **200** includes operations **202-210.** However, other embodiments can include additional or alternative operations or can omit one or more operations altogether. The process **200** is described as being executed by an analytics server, which can be the same as, or similar to, the analytics server **114a** described in **FIG. 1****.** However, one or more steps of the process **200** can be executed by any number of computing devices operating in the distributed computing system described in **FIG. 1****.** For instance, one or more computing devices can locally perform part, or all of the operations described in **FIG. 2****.**

At operation **202,** the analytics server can determine a first dose distribution indicating an instant amount of dose to be delivered to a planning target volume (PTV) of a patient. For example, the analytics server can determine a first dose distribution that indicates an instant amount of dose to be delivered to a PTV in accordance with a treatment plan. In some examples, the instant amount of dose can represent dose delivered to a PTV of a patient during a treatment session using a medical device such as a LINAC, proton beam generator, etc. The dose distribution delivered to the patient can represent a spatial pattern of radiation energy deposited within the patient's tissue. In an example, this distribution can be calculated and visualized as a two-dimensional and/or a three-dimensional map indicating how radiation dose varies throughout the PTV and surrounding OARs. In some examples, the dose distribution can reflect patient-specific factors such as tumor location, size, and shape, as well as facility-specific components like clinical goals and the medical device.

In some embodiments, the dose to be delivered to the PTV of the patient can be based on one or more treatment plan parameters. For example, a dose to be delivered to the PTV of the patient can be based on one or more treatment plan parameters represented by specific settings that are configurable during the radiotherapy planning process when generating treatment plans to configure medical devices to deliver dose within the PTV during a treatment session. In an example, these treatment plan parameters can include any combination of one or more of: beam arrangements, field sizes, beam energies, and modulation techniques that are used to shape the dose distribution. In this example, the treatment planning system can use these treatment plan parameters to calculate how energy will interact with the patient's anatomy and produce a three-dimensional map of dose that will be deposited within the PTV and surrounding tissue.

In an example, the analytics server can update (e.g., manipulate or change) the treatment plan parameters when targeting one or more clinical goals for PTV coverage. In this example, the analytics server can implement a treatment planning system as described herein to adjust treatment plan parameters corresponding to beam weighting, gantry angles, multileaf collimator positions, etc. to produce conformal dose distributions that closely match the shape of the PTV while minimizing dose to surrounding healthy tissues (OARs). In some examples, the analytics server can implement a treatment planning system that adjusts factors in accordance with advanced techniques like intensity-modulated radiation therapy (IMRT) to allow for greater control over the dose distribution by modulating the fluence of individual beamlets, allowing for more precise targeting of the PTV. The treatment plan parameters can be iteratively optimized to balance target coverage, dose homogeneity, and/or organ-at-risk sparing. In some embodiments, the analytics server can implement a reinforcement learning (RL) agent to update the treatment plan parameters as described herein.

In some embodiments, the analytics server can implement a machine learning model to generate the treatment plan. For example, the analytics server can implement an RL agent (also referred to as an "agent") to generate the treatment plan based on a patient profile. In an example, the analytics server can implement and train an RL agent that is configured to receive as input CT images of a patient representing the PTV and surrounding tissue, field geometry parameters, and/or prescription levels. These inputs can be represented by a patient profile indicating the patient when in a predetermined state (e.g., when scanned). In an example, the RL agent can be configured to operate a treatment planning system by iteratively adjusting the treatment plan parameters to optimize a dose distribution when targeting the PTV. In this example, the RL agent can update dose rate and multi-leaf collimator positions based on the current dose distribution and the configuration of the medical device. In some examples, the RL agent can learn to manipulate DVH constraints' locations and weights to achieve a treatment plan for each patient. In an example, the analytics server can also implement a tumor response model to estimate volume changes during treatment, allowing for continuous adaptation by the RL agent on a weekly or daily basis. In this example, the RL agent can learn to find optimal adaptation points and corresponding radiation doses. As a result, the RL agent can balance uncertainties in biological factors while maximizing tumor control and minimize toxicity to OARs. As the RL agent "learns," the RL agent can update a policy as described herein that is used to determine updates to subsequently-analyzed treatment plans. In an optional example, the policy of the RL agent may be trained to select optimal updates to treatment plans while optimizing tumor control and/or OAR toxicity.

At operation **204,** the analytics server can obtain a second dose distribution indicating a predetermined amount of dose delivered, or to be delivered, to the patient prior to the delivery of the first dose. For example, the analytics server can be configured to receive a second dose distribution indicating a treatment plan specifying energy delivered to the patient's PTV. This second dose distribution can be associated with a treatment plan that was generated when treating the patient at a time prior to the determination of the first dose distribution. For example, the second dose distribution can be associated with a historical treatment plan it was generated when treating the patient, where the historical treatment plan is included in a data set of one or more historical treatment plans corresponding to patients that were previously treated.

In some embodiments, the second dose distribution can indicate a predetermined amount of dose delivered to the patient. For example, the second dose distribution can indicate a predetermined amount of dose that was delivered to the patient when treated by a clinician. In some examples, the second dose distribution can be the product of a clinician configuring a treatment planning system to generate a treatment plan. For example, the second dose distribution can be the product of the clinician engaging with the treatment planning system and iteratively configuring and reconfiguring the treatment planning system until generating a treatment plan where a desired amount of dose is delivered to the PTV to be targeted with radiation.

At operation 206, the analytics server can determine a first result based on the first dose distribution and a second result for the second dose distribution. For example, the analytics server can provide the first dose distribution to a machine learning model (e.g., that is separate from the policy of the RL agent) to cause the machine learning model to generate an output. This output can represent a first outcome prediction. The first outcome prediction can indicate a probability associated with realization of a first off-target effect (e.g., a side effect as described herein) by the patient in response to receiving energy in accordance with the first dose distribution. The first off-target effect can include a side effect that is separate from an amount of dose that is measured as being delivered to the PTV or OARs of the patient. Similarly, the analytics server can provide the second dose distribution to the machine learning model to cause the machine learning model to generate an output. This output can represent a second outcome prediction. The second outcome prediction can indicate a probability associated with the first off-target effect associated with realization of the first off-target effect by the patient in accordance with the second dose distribution being delivered to the PTV or OARs of the patient. In both instances, the output of the ML model indicating the probability associated with the first off-target effect can be combined with a degree of an on-target effect achieved (e.g., DNA damage leading to cell death, protein modification, etc.) based on the dose delivery to generate a first result and a second result, respectively. While certain aspects of the present disclosure are discussed with respect to one side effect (including an off-target effect such as rectal bleeding, etc.), it will be understood that the ML model as described herein can be configured to determine a plurality of side effects as well as the probability that they will be realized by the patient when delivering dose in accordance with the corresponding dose distributions, allowing for the comparison of on-target effect and/or side effects/off-target effects on the patient when various treatment plans are implemented.

As described above, the ML model can be configured to receive a dose distribution (e.g., the first dose distribution and/or the second dose distribution) and generate a probability that one or more side effects will occur based on the energy delivered to the PTV and OARs. In an example, the ML model can be configured using historical patient data, including dose distributions and recorded toxicities, to learn patterns associated with specific side effects. In an optional example, the ML model may be trained to associate one or more side effects with energy delivered to the PTV and OARs. In this example, the ML model can include an artificial neural network (e.g., a convolutional neural network, recurrent neural networks, long short-term memory networks, etc.) that is configured to extract relevant features from the first dose distribution and/or the second dose distribution. In some examples, the ML model can incorporate additional patient-specific features like age, gender, and/or comorbidities to improve prediction accuracy. In an example, the ML model can process incoming dose distribution data in real-time during planning or treatment, calculating the probability of various toxicities based on the current delivered dose. In this example, the ML model can output risk scores for different side effects. In an optional example, the ML model may associate a risk score with each different type of side effect. In some examples, the output of the ML model can be integrated into the treatment planning system, providing immediate feedback on the likelihood of complications as planners adjust treatment plan parameters.

The analytics server can implement various types of ML models to generate predictions regarding side effects corresponding to dose distributions during radiotherapy. For example, the analytics server can implement convolutional neural networks (CNNs). In some examples, the analytics server can implement recurrent neural networks (RNNs), and/or long short-term memory (LSTM) networks if temporal aspects of the dose delivery are considered. Alternatively, feedforward neural networks with multiple hidden layers can be implemented by the analytics server.

The inputs to the ML model can include the dose distribution, which represents the spatial pattern of energy deposited within the PTV and surrounding OARs. Additional input features can include any combination of one or more of: patient-specific factors such as age, gender, tumor location, tumor size, and comorbidities. The outputs of the ML model can include probabilities of specific side effects occurring, such as rectal bleeding, radiation pneumonitis, xerostomia, etc. In some embodiments, the ML model can be configured to predict multiple side effects simultaneously or focus on a single complication or side effect. For instance, the ML model can be configured to predict both probabilities for the tumor control and the occurrence of a certain side-effect (e.g., rectal bleeding).

In some embodiments, the analytics server can train the ML model (e.g., by executing operations in accordance with one or more reinforcement learning agents, as described herein) by providing a dataset of historical dose distributions, recorded toxicities, etc., as an input to the ML model. During training, the ML model can process the input and generate one or more predictions. These predictions can be compared to the actual outcomes corresponding to the historical dose distributions using a loss function, such as binary cross-entropy. The analytics server can then update the weights of the ML model through backpropagation by implementing optimization algorithms like stochastic gradient descent. This process can be iteratively repeated over multiple epochs until the ML model's performance converges or reaches a threshold level of accuracy on a validation dataset of dose distributions and indications of off-target effects realized by the patients corresponding to the dose distributions. In an optional example, the ML model may be trained to generalize the historical data to a new patient.

In some embodiments, the analytics server can determine the first result based on the first dose distribution and a second result based on second dose distribution. For example, the analytics server can determine the first result by providing the first dose distribution to the ML model to cause the ML model to generate the first outcome prediction. Similarly, the analytics server can determine the second result by providing the second dose distribution to the ML model to cause the ML model to generate the second outcome prediction. The first outcome prediction can indicate a probability that is associated with a first off-target effect (e.g., a first side effect such as rectal bleeding, etc.) based on the dose delivery corresponding to the first dose distribution and the second outcome prediction can indicate a probability that is associated with the first off-target effect based on the dose delivery corresponding to the second dose distribution. The analytics server can then combine the first outcome prediction with a first degree of an on-target effect corresponding to the first dose distribution and the second outcome prediction with a second degree of the on-target effect corresponding to the second dose distribution to generate the first results and the second result, respectively.

At operation **208,** the analytics server can compare the first result and the second result to determine a difference between the first result and the second result. For example, to generate a first outcome prediction, the analytics server can provide the first dose distribution to the ML learning model as described above, prompting the ML model to generate a probability indicating whether, and to what degree, delivery of energy in accordance with the first dose distribution will result in the first off-target effect impacting the health of the patient. The second dose distribution can similarly be provided to the same ML model to cause the ML model to produce a second outcome prediction. The second outcome prediction can similarly indicate a probability as to whether, and to what degree, the second dose distribution will result in the first off-target effect impacting the health of the patient. The analytics server can then compare these two predictions as well as the corresponding degrees of on- and off-target effect(s) achieved by the first dose distribution and the second dose distribution to determine a difference between the first result and the second result. This comparison can involve comparing the degree of on- and/or off-target effect associated with the first dose distribution and the second dose distribution as well as a comparison of the degree to which the first off-target effect will impact the patient. By comparing these two predictions, the analytics server can determine the difference between the first result and second result, where this difference signifies how much the first result diverges from the second.

At operation **210,** the analytics server can execute an update to the one or more treatment plan parameters (e.g., using a policy configured by an RL agent as described below) based on the difference between the first result and the second result, described above. For example, the analytics server can execute the update to the one or more treatment plan parameters based on the difference between the first result and the second result (such as the difference between the dose distributions associated with the first result and the second result, respectively). In some embodiments, the analytics server can execute the update to configure adjustment of the operation of a medical device as described herein in accordance with the one or more treatment plan parameters described herein. For example, the analytics server can determine that a dose distribution resulted in a higher degree of on-target effect to the PTV, but also resulted in a higher probability that the patient will experience one or more off-target effects where the probability extends beyond corresponding probability thresholds for each side effect. As described herein, the analytics server can configure an RL agent to determine the update to the one or more treatment plan parameters based on training of the RL agent as described herein. In an optional example, the RL agent may determine the update to the one or more treatment plan parameters based on the trained policy of the RL agent.

In some embodiments, the RL agent can be configured to receive multiple inputs related to dose distributions and their associated side effects that are associated with the development of a treatment plan for a patient. In an example, the RL agent can be configured to receive a first dose distribution and a second dose distribution as inputs, representing different potential treatment approaches. In this example, the RL agent can also be configured to receive a first result indicating the likelihood of the patient experiencing a specific side effect when the first dose distribution is applied. In some examples, the RL agent can also be configured to receive a second result indicating the likelihood of the patient experiencing the same side effect when the second dose distribution is applied. In an example, the RL agent can process these inputs using its trained model (also referred to as a policy) to evaluate the trade-offs between the different dose distributions and their associated side effect risks, such as trade-offs between PTV dose and side effect risks. In this example, based on its analysis of the provided inputs, the RL agent can be configured to generate an output indicating one or more changes to the treatment plan parameters. In some examples, these changes can include adjustments to the dose parameters, modifications to the treatment schedule, and/or alterations to other relevant parameters that may help optimize the balance between treatment efficacy and side effect minimization.

In some embodiments, the analytics server can implement the policy referred to above (e.g., a machine learning model) as the RL agent to generate updates to treatment plan parameters. In an example, the model can include an artificial neural network architecture with multiple hidden layers to approximate a function f(x) (sometimes referred to as a Q-function) that estimates the expected cumulative reward for applying different treatment plan parameters for patient states. The RL agent can receive as inputs the first dose distribution, second dose distribution, the first result indicating the likelihood of side effects for the first distribution, and second result indicating the likelihood of side effects for the second distribution. In an example, these inputs can be encoded as a state vector representing the patient's current condition and treatment options. In this example, the RL agent can be configured to output a set of recommended changes to treatment plan parameters. For example, the set of recommended changes to the treatment plan parameters can include adjustments to any combination of one or more of: dose levels, power levels, beam configurations at certain control points, beam angles at the corresponding control points, etc. In some examples, the output can be discretized into a finite set of possible actions to simplify the action space.

In some embodiments, the analytics server can train the RL agent. For example, the analytics server can use historical patient data, simulated treatment scenarios, or a combination of historical patient data and simulated treatment scenarios. In an example, each training episode can consist of a sequence of state-action-reward tuples, where the state is the patient's condition and current treatment plan, the action is a proposed change to the plan parameters, and the reward is a function of treatment efficacy and/or side effect mitigation. In some examples, the RL agent policy can be iteratively updated by selecting actions that are configured to result in treatment plans that balance exploitation of known good actions (e.g., increased dose delivery such as increased dose delivery to a target and/or on-target effect, reduced probabilities of side effects being experienced, etc.) with exploration of potentially better alternatives. In an example, when the RL agent receives a positive reward for an action that improves treatment outcomes and/or reduces side effects, reward for state-action pair can be increased, making the action more likely to be selected in similar future situations. Conversely, for an action that does not improve treatment outcomes and/or reduce side effects, the RL agent can receive a penalty that can lead to decreased rewards, thereby discouraging their future selection of similar actions. The analytics server can iteratively update and test the policy of the RL agent until the policy converges (e.g., the treatment plan parameters result in the delivery of energy to the PTV and OARs that satisfies one or more treatment plan parameters while also satisfying one or more thresholds indicating acceptable probabilities that the side effects will be realized by the patient). For example, the analytics server can iteratively update the policy of the RL agent until a predetermined degree of on-target effect is satisfied as well as a predetermined degree corresponding to a probability that the first side effect, or an off-target effect, will be realized by the patient is satisfied.

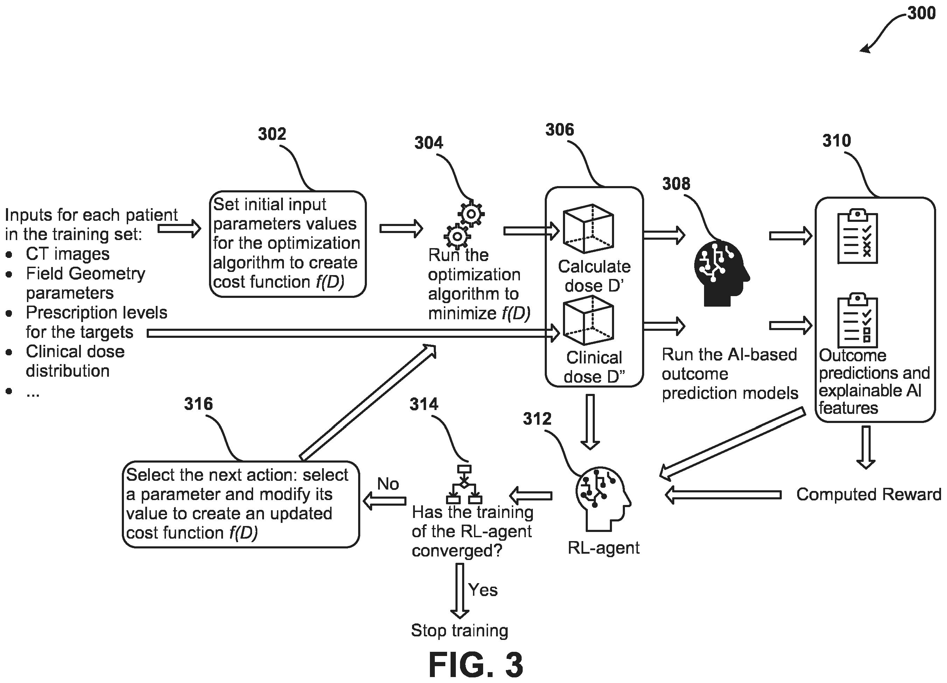

**FIG. 3** illustrates an implementation diagram **300** of a process for learning a treatment policy by training a reinforcement learning agent for radiotherapy treatment plan generation, according to an embodiment. In some embodiments, one or more aspects of the implementation of the process represented by the implementation diagram **300** can be implemented by an analytics server such as the analytics server **114a** of **FIG. 1****.**

As shown by the implementation diagram **300** of **FIG. 3****,** an RL agent **312** can be used to learn a policy (e.g., a model as described herein) to control (e.g., steer) the treatment plan generation process. As an example, an RL agent **312** can be implemented during the treatment plan optimization process to configure a policy (e.g., a model, a function, etc.) that maps a state of a patient to a treatment plan. The RL agent **312** can evaluate the on- and off-target effects of treatment plans generated by the RL agent **312** on treatment outcomes by comparing the treatment outcome of a given treatment plan to the treatment outcome of other treatment plans generated by the RL agent **312.** The RL agent **312** can then be rewarded or penalized based on the treatment plans generated by the RL agent **312.** As a result, the RL agent **312** can be configured (e.g., can "learn") through the generation of multiple treatment profiles and by obtaining reward signals to optimize cumulative rewards. Once the policy is fully established by the RL agent **312,** the RL agent 312 may no longer update the policy in accordance with the reward signal.

In some examples, an RL agent **312** can interact with a plan optimization algorithm (e.g., implemented by a treatment planning system as described herein) by changing treatment plan parameters, including optimization objectives' locations and/or weights, and/or field geometry information. At each iteration, the RL agent **312** can modify one or more of the treatment plan parameters and execute a plan optimization algorithm. The optimization can continue until a maximum number of iterations are reached or convergence criteria are satisfied (e.g., prescribed dose is delivered to a threshold portion of the PTV, conformance of dose delivery with the PTV is established within a threshold degree, and/or the like). New observations (e.g., optimized 3D dose distributions) and reward values can be provided to the RL agent **312.** During training, the RL agent **312** can access patient data associated with the patient for which a treatment plan is being processed, including CT images, structure sets, and/or clinical plans with associated dose distributions manually created and accepted for treatment. The training process can involve setting initial optimization input parameters, triggering the optimization algorithm, returning optimized dose distributions as observations, and receiving outcome predictions for both optimized and clinical dose distributions. Explainable components, such as gradient-weighted Class Activation Mapping (Grad-CAM) heatmaps, can be included in the observations.

The reward returned to the RL agent **312** can be determined by comparing predictions given by outcome prediction models **308** executed on optimized and clinical doses. Initially, the reward scheme can be biased to achieve similar outcomes to a manual plan for both tumor control (e.g., control of the growth of the PTV) and healthy tissue sparing (reduction of the off-target effects realized by the surrounding OARs). The RL agent **312** can then receive positive rewards for further improving outcomes, such as better healthy tissue sparing while maintaining tumor control. The reward can also include a measure of efficiency, defined as the number of times the RL agent **312** triggers the optimizer before generating a clinically acceptable plan with desired treatment outcomes. Based on returned observations, rewards, and its current policy, the RL agent **312** can decide its next action, selecting an input parameter and proposing a new value for the input parameter. The newly modified input parameter can be provided for a new round of optimization, repeating the process until the RL agent **312** converges.

With continued reference to implementation diagram **300,** to begin the optimization process, at operation **302** an analytics server can obtain initial values of input parameters, such as the locations and weights of the optimization objectives and the field geometry parameters. This can be done using a template established for similar treatment plans. Once the input parameters are established, at operation **304** the analytics server can execute an optimization algorithm. At operation **306** an optimized dose distribution D' can be returned as an observation to the RL agent **312,** along with AI-based outcome predictions for both the optimized dose D' and a clinical dose distribution D" established based on an earlier-developed treatment plan for the patient, covering tumor control and healthy tissue sparing. In examples where explainable AI components are available for the outcome prediction models **308** (which can be the same as, or similar to, the models described herein that are configured to determine one or more off-target effects that result from a given dose distribution), these features, such as Grad-CAM heatmaps corresponding to the dose distributions indicating the most influential input image features (e.g., regions where dose was delivered effectively to achieve on-target effect in the PTV), can also be included in the observation.

In some implementations, at operation **310** a reward for the RL agent **312** can be derived from comparing the predictions of the outcome models for the optimized dose and the clinical dose from the manual plan. Initially, a reward scheme can favor achieving outcomes similar to those of the manual plan for both tumor control and healthy tissue sparing. Once this is achieved, the RL agent **312** can continue to receive positive rewards if the RL agent **312** further improves the outcomes, such as better healthy tissue sparing while maintaining tumor control results. Additionally, the reward can include a measure of efficiency, indicated by the number of times the RL agent **312** triggers the optimizer before generating a clinically acceptable plan with the desired treatment outcomes.

Based on the observations and rewards received, and its current policy, at operation **316** the RL agent **312** can decide a next action by choosing one of the input parameters and proposing a new value for it. The newly modified input is then provided for a new round of optimization. In some examples, at operation **314,** when the analytics server determines that the policy being trained in response to actions taken by the RL agent **312** has converged, the analytics server can stop training of the policy by the RL agent **312.**

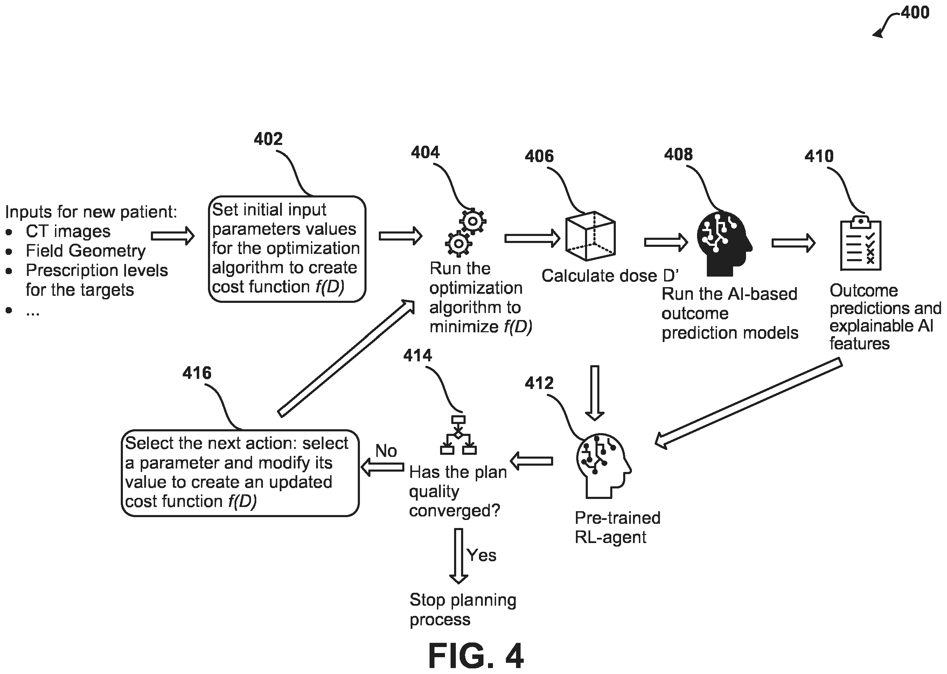

Referring to **FIG. 4****,** illustrated is an implementation diagram **400** of a process for using a policy trained using a reinforcement learning agent when generating radiotherapy treatment plans, according to an embodiment. In some embodiments, one or more aspects of the implementation of the process represented by the implementation diagram **400** can be implemented by an analytics server such as the analytics server **114a** of **FIG. 1****.**

To begin the optimization process, at operation **402,** the analytics server can configure the initial values of the input parameters, such as the locations and weights of the optimization objectives and the field geometry parameters. Similar to above, this can be done using a template. Once these parameters are established, at operation **404** the analytics server can execute the optimization algorithm. The optimization algorithm can process the input parameters and returns an optimized dose distribution D' **406** as an observation to the RL agent **412.** Along with this optimized dose distribution D', one or more AI based outcome prediction models **408** can be executed. That operation **410,** one or more AI-based outcome predictions can also be provided indicating whether or not one or more on- or off-target effects are likely to be experienced by the patient in response to delivery of energy in accordance with the optimized dose distribution D' **406.** These predictions can include assessments for either or both tumor control and healthy tissue sparing, using the corresponding models described herein. If explainable AI components are available for the outcome prediction models, their outputs are also included as part of the observations.

At operation **416,** the RL agent **412,** based on its trained policy, can select one of the input parameters and modify its value to create a new cost function configured to achieve a better treatment outcome. This process of triggering the optimization algorithm, returning the optimized dose distribution and outcome predictions, and modifying the input parameters can be repeated iteratively. At operation **414,** convergence can be reached where no further improvement, or where no further significant improvement, in the predicted treatment outcomes can be achieved. One advantage with this approach is that the analytics server can also use the predicted dose distribution during the inference process, as a guideline for the RL agent for the minimal level of plan quality we aim to achieve.

The various illustrative logical blocks, modules, circuits, and algorithm steps described in connection with the embodiments disclosed herein can be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans can implement the described functionality in varying ways for each particular application, but such implementation decisions should not be interpreted as causing a departure from the scope of this disclosure or the claims.

Embodiments implemented in computer software (e.g., computer programs, computer program products, etc.) can be implemented in software, firmware, middleware, microcode, hardware description languages, or any combination thereof. A code segment or machine-executable instructions can represent a procedure, a function, a subprogram, a program, a routine, a subroutine, a module, a software package, a class, or any combination of instructions, data structures, or program statements. A code segment can be coupled to another code segment or a hardware circuit by passing or receiving information, data, arguments, parameters, or memory contents. Information, arguments, parameters, data, etc., can be passed, forwarded, or transmitted via any suitable means, including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions can be stored as one or more instructions or code on a non-transitory computer-readable or processor-readable storage medium. The steps of a method or algorithm disclosed herein can be embodied in a processor-executable software module, which can reside on a computer-readable or processor-readable storage medium. A non-transitory computer-readable or processor-readable media includes both computer storage media and tangible storage media that facilitate the transfer of a computer program from one place to another. A non-transitory processor-readable storage media can be any available media that can be accessed by a computer. By way of example, and not limitation, such non-transitory processor-readable media can comprise RAM, ROM, EEPROM, CD-ROM or other optical disk storage, magnetic disk storage or other magnetic storage devices, or any other tangible storage medium that can be used to store desired program code in the form of instructions or data structures and that can be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm can reside as one or any combination or set of codes or instructions on a non-transitory processor-readable medium or computer-readable medium, which can be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein can be applied to other embodiments without departing from the scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1. A system for efficient policy generation using reinforcement learning to optimize radiotherapy treatment plan generation, the system comprising:
one or more processors configured to:
obtain a profile of a patient in a predetermined state, the profile of the patient indicating one or more locations for tissue to be targeted;
provide the profile of the patient as input to a policy configured using a reinforcement learning (RL) agent to cause the policy to generate an output in accordance with one or more treatment plan parameters,
wherein the RL agent is configured to iteratively update the policy based on dose distributions and corresponding on-target and off-target effects resulting from targeting tissue during radiotherapy treatment; and
control operation of a medical device based on the output of the policy.

2. The system of claim 1, wherein the off-target effects comprise one or more of: rectal bleeding, radiation pneumonitis, or xerostomia and wherein the on-target effects comprise one or more of: local control of a tumor or tumor recurrence.

3. The system of claim 1 or claim 2, wherein the one or more processors are further configured to:
generate one or more control signals to control operation of the medical device in accordance with the output of the policy,
wherein the one or more processors configured to control operation of the medical device are configured to:
control operation of the medical device based on the one or more control signals; and/or:
wherein the medical device comprises a linear accelerator (LINAC), and
wherein the one or more processors configured to control operation of the medical device are configured to:
configure the medical device to deliver dose to at least a portion of the tissue to be targeted based on the output of the policy.

4. A system comprising:
one or more processors configured to:
determine a first dose distribution indicating an instant amount of dose to be delivered to a planning target volume (PTV) of a patient at a first point in time based on one or more treatment plan parameters that control operation of a linear accelerator (LINAC);
obtain a second dose distribution indicating a predetermined amount of dose delivered to the patient during treatment at a second point in time prior to the first point in time;
determine a first result based on the first dose distribution and a second result for the second dose distribution, the first result indicating a first degree of an on-target effect and a first off-target effect, and the second result indicating a second degree of on-target effect and a second off-target effect;
compare the first result and the second result to determine a difference between the first result and the second result; and
execute an update to the one or more treatment plan parameters based on the difference between the first result and the second result,
wherein the update is configured to adjust operation of a linear accelerator (LINAC) in accordance with the one or more treatment plan parameters.

5. The system of claim 4, wherein the one or more processors are further configured to:
obtain a profile representing a patient in a predetermined state having tissue to be targeted using the treatment plan parameters; and
determine the one or more treatment plan parameters based on the profile of the patient in the predetermined state;
and optionally:
wherein the profile comprises one or more computed tomography (CT) images, one or more field geometry parameters, and one or more prescription levels indicating a target dose to deliver to the PTV of the patient.

6. The system of claim 4 or claim 5, wherein the one or more processors configured to compare the first result and the second result are configured to:
provide the first dose distribution to a machine learning model to cause the machine learning model to generate a first outcome prediction indicating a probability associated with the first off-target effect and the first on-target effect;
provide the second dose distribution to the machine learning model to cause the machine learning model to generate a second outcome prediction indicating a probability associated with the second off-target effect; and
compare the first outcome prediction to the second outcome prediction to determine the difference between the first result and the second result, where the difference indicates a degree according to which the first result is different from the second result;
and optionally:
wherein the one or more processors configured to provide the first dose distribution to the machine learning model are configured to:
provide the first dose distribution to the machine learning model to cause the machine learning model to generate a first outcome prediction indicating a first probability that the patient will experience one or more off-target effects and one or more on-target effects, and
provide the second dose distribution to the machine learning model to cause the machine learning model to generate a second outcome prediction indicating a second probability that the patient will experience the one or more off-target effects.

7. The system of any of claim 4 to claim 6, wherein the one or more processors are further configured to:
provide the first dose distribution, the second dose distribution, the first result, and the second result to a reinforcement learning (RL) agent to cause the RL agent to determine the update to the one or more treatment plan parameters;
and optionally:
(i) wherein the one or more processors are further configured to:
determine that updating the treatment plan parameters causes one or more of the treatment plan parameters to satisfy a convergence threshold; and
determine that the RL agent converged based on the treatment plan parameters satisfying the convergence threshold;
and/or
(ii) wherein the one or more processors are further configured to:
determine that one or more of the treatment plan parameters do not satisfy a convergence threshold based on updating the treatment plan parameters; and
in response to determining that the one or more treatment plan parameters do not satisfy the convergence threshold, cause the RL agent to update a policy to adjust outputs of the policy based on the treatment plan parameters not satisfying the convergence threshold.

8. The system of any of claim 4 to claim 7, wherein the one or more processors configured to determine the first dose distribution indicating an instant amount of dose delivered to a planning target volume (PTV) of a patient based on one or more treatment plan parameters are configured to:
determine the first dose distribution indicating an instant amount of dose delivered to a planning target volume (PTV) of a patient based on a cost function establishing the one or more treatment plan parameters.

9. A method, comprising:
determining, by one or more processors, a first dose distribution indicating an instant amount of dose to be delivered to a planning target volume (PTV) of a patient at a first point in time based on one or more treatment plan parameters that control operation of a linear accelerator (LINAC);
obtaining, by the one or more processors, a second dose distribution indicating a predetermined amount of dose delivered to the patient during treatment at a second point in time prior to the first point in time;
determining, by the one or more processors, a first result based on the first dose distribution and a second result for the second dose distribution, the first result indicating a first degree of on-target effect and a first off-target effect, and the second result indicating a second degree of on-target effect and a second off-target effect;
comparing, by the one or more processors, the first result and the second result to determine a difference between the first result and the second result; and
executing, by the one or more processors, an update to the one or more treatment plan parameters based on the difference between the first result and the second result,
wherein the update is configured to adjust operation of a linear accelerator (LINAC) in accordance with the one or more treatment plan parameters.

10. The method of claim 9, further comprising:
obtaining, by the one or more processors, a profile representing a patient in a predetermined state having tissue to be targeted using the treatment plan parameters; and
determining, by the one or more processors, the one or more treatment plan parameters based on the profile of the patient in the predetermined state;
and optionally:
wherein the profile comprises one or more computed tomography (CT) images, one or more field geometry parameters, and one or more prescription levels indicating a target dose to deliver to the PTV of the patient.

11. The method of claim 9 or claim 10, wherein comparing the first result and the second result comprises:
providing, by the one or more processors, the first dose distribution to a machine learning model to cause the machine learning model to generate a first outcome prediction indicating a probability associated with the first off-target effect;
providing, by the one or more processors, the second dose distribution to the machine learning model to cause the machine learning model to generate a second outcome prediction indicating a probability associated with the second off-target effect; and
comparing, by the one or more processors, the first outcome prediction to the second outcome prediction to determine the difference between the first result and the second result, where the difference indicates a degree according to which the first result is different from the second result;
and optionally:
wherein providing the first dose distribution to the machine learning model comprises:
providing, by the one or more processors, the first dose distribution to the machine learning model to cause the machine learning model to generate a first outcome prediction indicating a first probability that the patient will experience one or more off-target effects, and
providing, by the one or more processors, the second dose distribution to the machine learning model to cause the machine learning model to generate a second outcome prediction indicating a second probability that the patient will experience the one or more off-target effects.

12. The method of any of claim 9 to claim 11, further comprising:
providing, by the one or more processors, the first dose distribution, the second dose distribution, the first result, and the second result to a reinforcement learning (RL) agent to cause the RL agent to determine the update to the one or more treatment plan parameters;
and optionally:
determining, by the one or more processors, that updating the treatment plan parameters causes one or more of the treatment plan parameters to satisfy a convergence threshold; and
determining, by the one or more processors, that the RL agent converged based on the treatment plan parameters satisfying the convergence threshold.

13. The method of any of claim 9 to claim 12, wherein determining the first dose distribution indicating an instant amount of dose delivered to a planning target volume (PTV) of a patient based on one or more treatment plan parameters comprises:
determining, by the one or more processors, the first dose distribution indicating an instant amount of dose delivered to a planning target volume (PTV) of a patient based on a cost function establishing the one or more treatment plan parameters.

14. A non-transitory computer-readable medium storing instructions thereon that, when executed by one or more processors, cause the one or more processors to:
determine a first dose distribution indicating an instant amount of dose to be delivered to a planning target volume (PTV) of a patient at a first point in time based on one or more treatment plan parameters that control operation of a linear accelerator (LINAC);
obtain a second dose distribution indicating a predetermined amount of dose delivered to the patient during treatment at a second point in time prior to the first point in time;
determine a first result based on the first dose distribution and a second result for the second dose distribution, the first result indicating a first degree of on-target effect and a first off-target effect, and the second result indicating a second degree of on-target effect and a second off-target effect;
compare the first result and the second result to determine a difference between the first result and the second result; and
execute an update to the one or more treatment plan parameters based on the difference between the first result and the second result,
wherein the update is configured to adjust operation of a linear accelerator (LINAC) in accordance with the one or more treatment plan parameters.

15. The non-transitory computer-readable medium of claim 14, wherein the instructions further cause the one or more processors to:
obtain a profile representing a patient in a predetermined state having tissue to be targeted using the treatment plan parameters; and
determine the one or more treatment plan parameters based on the profile of the patient in the predetermined state;
and optionally:
wherein the profile comprises one or more computed tomography (CT) images, one or more field geometry parameters, and one or more prescription levels indicating a target dose to deliver to the PTV of the patient.
